# EUROPÄISCHE PATENTANMELDUNG

(11) **EP 3 375 405 A1**
(43) Veröffentlichungstag der Anmeldung: **19.09.2018**
(21) Anmeldenummer: 17161599.0
(22) Anmeldetag: 17.03.2017
(51) Int. Cl.: A61C 9/00

(54) **VORRICHTUNG ZUM BETREIBEN EINES INTRAORALSCANNERS**

(71) Anmelder: a.tron3d GmbH, 9020 Klagenfurt am Wörthersee (AT)
(72) Erfinder: JESENKO, Juergen, 9587 Riegersdorf (AT)
(74) Vertreter: Beer & Partner Patentanwälte KG

(57) **Zusammenfassung**

Bei einer Vorrichtung zum Betreiben eines Intraoralscanners mit wenigstens einem Handstück (1) und wenigstens einer Recheneinheit sind das Handstück (1) und die Recheneinheit (2) voneinander elektrisch isoliert.

## Beschreibung

Die Erfindung betrifft eine Vorrichtung zum Betreiben eines Intraoralscanners mit wenigstens einem Handstück und wenigstens einer Recheneinheit.

In der Zahntechnik ist es für viele Aufgaben erforderlich, ein Modell der Zähne und auch anderer intraoraler Strukturen zu haben. Wurden solche Modelle früher noch physisch durch Zahnabdrücke erzeugt und beispielweise als Gipsmodelle aufbewahrt, ist es heute mehr und mehr üblich, derartige Modelle virtuell zu erfassen und zu speichern. Damit kann nicht nur der Raum für das Aufbewahren der Modelle vermindert werden, sondern es wird dem Patienten auch das unangenehme Abnehmen des Zahnbadruckes erspart.

Hinsichtlich ihrer Hardware weisen Intraoralscanner zwei wesentliche Einheiten auf: Handstück und Recheneinheit. Dabei hat sich gezeigt, dass die beiden Einheiten mit einer physischen Verbindung zu Kommunikation verbunden sind, da die zum Teil sehr großen Datenmengen, welche beim Scannen entstehen können, in der gewünschten Geschwindigkeit kommuniziert werden können. Während die Recheneinheit theoretisch an einem beliebigen Ort angeordnet sein kann, kommt das Handstück unweigerlich mit dem Patienten, dessen virtueller Zahnabdruck genommen werden soll, in Berührung.

Kommen elektrisch betriebene Geräte in medizinischen Bereichen zum Einsatz, müssen besondere Maßnahmen zum Schutz des Anwenders (MOOP, "Means of Operator Protection") oder des Patienten (MOPP, "Means of Patient Protection") getroffen werden, wobei der Schutz des Patienten automatisch den Schutz des Anwenders beinhaltet. Diese Schutzmaßnahmen werden üblicherweise durch besondere Netzteile getroffen, die allerdings für Geräte mit hohem Energiebedarf sehr kostspielig sind und damit die Kosten für das gesamte Gerät stark erhöhen.

Der Erfindung liegt daher die Aufgabe zu Grunde, diesen Nachteil zu überwinden und eine kostengünstige Methode zur Verfügung zu stellen, Intraoralscanner für Anwender und Patienten sicher betreiben zu können.

Gelöst wird diese Aufgabe erfindungsgemäß durch eine Vorrichtung der eingangs genannten Art, die dadurch gekennzeichnet ist, dass das Handstück und die Recheneinheit voneinander elektrisch isoliert sind.

Durch das elektrische Isolieren von Handstück und Recheneinheit wird die Möglichkeit geschaffen, unterschiedliche Stromquellen für die Recheneinheit und das Handstück zu verwenden. Bevorzugt weist das Handstück daher eine eigene Energiequelle auf. Diese ist bevorzugt ein eigenes Netzteil. Da das Handstück eines Intraoralscanners üblicherweise selbst nur einen sehr geringen Energiebedarf hat, kann hierfür ein einfaches und kostengünstiges Netzteil für die Verwendung in einem medizinischen Umfeld verwendet werden.

Alternativ kann das Handstück auch mit einem Energiespeicher als Stromquelle ausgestattet sein. Hierfür kann eine Ladestation vorgesehen sein, in der das Handstück gelagert wird, wenn es nicht benutzt wird.

Um trotz der elektrischen Isolierung von Handstück und Recheneinheit eine Datenübertragung zwischen den beiden Einheiten des Intraoralscanners zu ermöglichen, ist gemäß einer bevorzugten Ausführungsform der Erfindung vorgesehen, dass das Handstück und die Recheneinheit mit einer nicht galvanischen Verbindung zum Übertragen von Daten verbunden sind.

Bevorzugt ist die nicht elektrische Verbindung zum Übertragen von Daten ein Lichtleiter, da dabei besonders große Datenübertragungsraten erzielt werden können.

In einer bevorzugten Ausführungsform der Erfindung weist die Verbindung zum Übertragen von Daten wenigstens zwei Teilbereiche auf. In einer bevorzugten Weiterbildung ist wenigstens ein Teilbereich eine nicht galvanische Verbindung. Gemäß einer weiteren bevorzugten Weiterbildung der Erfindung kann dies insbesondere ein Lichtleiter sein.

Durch eine Teilung des Lichtleiters in vorzugsweise seriell aufeinanderfolgende Teilbereiche oder -abschnitte können die verschiedenen Vorteile möglicher Übertragungswege und Verbindungen genutzt werden. So kann beispielsweise die elektrische Isolierung über einen Lichtleiter realisiert werden, für einen anderen Teilbereich jedoch die größere Flexibilität und die variableren Einsatzmöglichkeiten von metallischen Leitern genutzt werden.

Weiters sind häufig bereits optische oder auch galvanische Leitungen in zahnärztlichen Behandlungsstühlen verlegt. Durch eine Kombination verschiedener und gegebenenfalls verschiedenartiger Abschnitte bzw. Teilbereiche kann die Vorrichtung einfach in einen solchen Behandlungsstuhl integriert werden.

Dementsprechend ist gemäß bevorzugter Weiterbildungen der Erfindung vorgesehen, dass wenigstens ein Teilbereich der Verbindung oder auch die Recheneinheit Teil eines zahnärztlichen Behandlungsstuhls ist.

Weitere bevorzugte Ausführungsformen der Erfindung sind Gegenstand der Unteransprüche. Nachstehend ist ein bevorzugtes, jedoch den Schutzbereich der Anmeldung nicht beschränkendes, Ausführungsbeispiel der Erfindung anhand der Zeichnung näher beschrieben. Es zeigt:

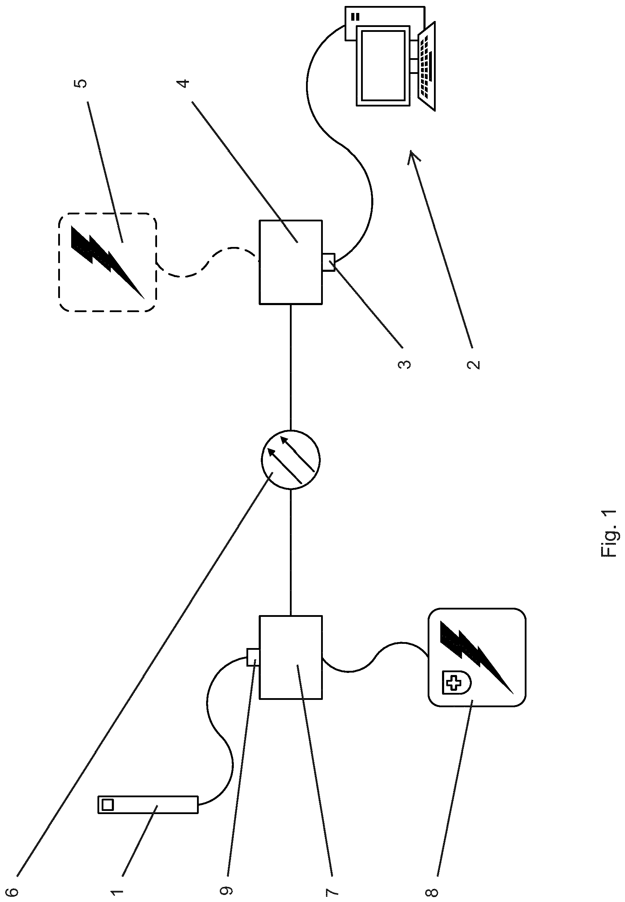

- Fig. 1: schematisch einen möglichen Aufbau einer erfindungsgemäßen Vorrichtung.

Die in Fig. 1 gezeigte Vorrichtung zum Betreiben eines Intraoralscanners weist ein Handstück 1 und eine Recheneinheit 2 auf.

In der dargestellten Ausführungsform geht von der Recheneinheit 2 eine rechnerseitige USB-Verbindung 3 zu einem rechnerseitigen Transceiver 4. Selbstverständlich sind auch andere Verbindungen für die Kommunikation mit einem Computer denkbar, für den Betrieb von Intraoralscannern hat sich die Verwendung einer USB 3.0 Schnittstelle allerdings gut bewährt, da diese neben der gewünschten Kommunikationsbandbreite auch eine ausreichende Energieversorgung bieten kann. Sollte eine Datenverbindung gewählt werden, die nicht in der Lage ist, eine ausreichende Energieversorgung zur Verfügung zu stellen, kann optional zusätzlich ein Netzgerät 5 am rechnerseitigen Transceiver 4 angeschlossen sein. Hierfür kann ein handelsübliches Netzgerät ohne besondere, für den Einsatz in der Medizintechnik vorgesehene Sicherheitsvorkehrungen verwendet werden.

Im rechnerseitigen Transceiver 4 werden dann von der Recheneinheit 2 kommende Steuersignale in optische Signale 6 umgewandelt und über eine optische Verbindung an einen zweiten, handstückseitigen Transceiver 7 weitergeleitet.

Der handstückseitige Transceiver 7 wird von einer geschützten und für den Gebrauch in einem medizinischen Kontext geeigneten (MOOP) Sicherheitsnetzteil 8 mit Energie versorgt. Im handstückseitigen Transceiver 7 wird das optische Signal 6 empfangen und wieder in ein elektrisches Signal umgewandelt. Dabei kann durch das Kombinieren von Daten und Stromversorgung beispielsweise ein USB-Signal erzeugt werden.

In der dargestellten Ausführungsform wird das kombinierte Signal über einen handstückseitigen USB-Anschluss 9 weitergegeben. Dieser dient auch dazu, das Handstück mit Energie zu versorgen.

Durch die erfindungsgemäße Vorrichtung können eine Recheneinheit 2 und ein Handstück 1 nach dem Stand der Technik verwendet werden, ohne dass sie selbst für den elektrisch sicheren Gebrauch in einem medizinischen Kontext modifiziert werden müssen.

Ein weiterer Vorteil der erfindungsgemäßen Vorrichtung ergibt sich, wenn ein Intraoralscanner in einen bestehenden Behandlungsstuhl integriert werden soll, da so nur der Raum für das Sicherheitsnetzteil 8, den handstückseitigen Transceiver 7 und die Verkabelung benötigt wird, wobei die Recheneinheit 2 und gegebenenfalls auch der rechnerseitige Transceiver räumlich getrennt stehen können.

### Bezugszeichenliste:

- 1: Handstück
- 2: Recheneinheit
- 3: USB (rechnerseitig)
- 4: Transceiver (rechnerseitig)
- 5: Netzgerät (normal)
- 6: optische(s) Signal(e)
- 7: Transceiver (handstückseitig)
- 8: Sicherheitsnetzteil
- 9: USB (handstückseitig)

## Patentansprüche

1. Vorrichtung zum Betreiben eines Intraoralscanners mit wenigstens einem Handstück (1) und wenigstens einer Recheneinheit (2), **dadurch gekennzeichnet, dass** das Handstück (1) und die Recheneinheit (2) voneinander elektrisch isoliert sind.

2. Vorrichtung nach Anspruch 1, **dadurch gekennzeichnet, dass** das Handstück (1) eine Energiequelle aufweist.

3. Vorrichtung nach Anspruch 2, **dadurch gekennzeichnet, dass** die Energiequelle ein Sicherheitsnetzteil (8) ist.

4. Vorrichtung nach Anspruch 2, **dadurch gekennzeichnet, dass** die Energiequelle ein Energiespeicher ist.

5. Vorrichtung nach einem der Ansprüche 1 bist 4, **dadurch gekennzeichnet, dass** das Handstück (1) und die Recheneinheit (2) mit einer nicht galvanischen Verbindung zum Übertragen von Daten verbunden sind.

6. Vorrichtung nach Anspruch 5, **dadurch gekennzeichnet, dass** die Verbindung zum Übertragen von Daten ein Lichtleiter ist.

7. Vorrichtung nach Anspruch 5 oder 6, **dadurch gekennzeichnet, dass** die Verbindung zum Übertragen von Daten wenigstens zwei Teilbereiche aufweist.

8. Vorrichtung nach Anspruch 7, **dadurch gekennzeichnet, dass** wenigstens ein Teilbereich eine nicht galvanische Verbindung ist.

9. Vorrichtung nach Anspruch 7 oder 8, **dadurch gekennzeichnet, dass** wenigstens ein Teilbereich ein Lichtleichter ist.

10. Vorrichtung nach einem der Ansprüche 7 bis 9, **dadurch gekennzeichnet, dass** wenigstens ein Teilbereich Teil eines zahnärztlichen Behandlungsstuhls ist.

11. Vorrichtung nach einem der Ansprüche 1 bis 10, **dadurch gekennzeichnet, dass** die Recheneinheit (2) Teil eines zahnärztlichen Behandlungsstuhls ist.
